# FASCICULE DE BREVET EUROPEEN

(11) **EP 1 400 531 B1**
(45) Date de publication et mention de la délivrance du brevet: **04.01.2006**
(21) Numéro de dépôt: 03292405.2
(22) Date de dépôt: 30.09.2003
(51) Int. Cl.: C07K 5/06, C07C 227/32, C07K 5/02, C07D 209/42

(54) **Procédé de synthèse de la N-[(S)-1-carbéthoxybutyl]-(S)-alanine et application à la synthèse du perindopril**
Verfahren zur Herstellung von N-((S)-1-(Etoxycarbonyl)butyl)-(S)-alanin und Verwendung in der Synthese von Perindopril
Process for the synthesis of N-((S)-1-(ethoxycarbonyl)butyl)-(S)-alanine and use in the synthesis of perindopril

(43) Date de publication de la demande: 24.03.2004
(73) Titulaire: Les Laboratoires Servier, 92415 Courbevoie Cedex (FR)
(72) Inventeur: Breard, Fabienne, 76140 Petit Quevilly (FR); Lecouve, Jean-Pierre, 76600 Le Havre (FR)

(56) Documents cités:
- WO-A-01/56353
- WO-A-01/56972
- BENNION C ET AL: "Design, synthesis, and physicochemical properties of a novel, conformationally restricted 2,3-dihydro-1,3,4-thiadiazole-containing angiotensin converting enzyme inhibitor which is preferentially eliminated by the biliary route in rats" JOURNAL OF MEDICINAL CHEMISTRY, AMERICAN CHEMICAL SOCIETY. WASHINGTON, US, vol. 34, no. 1, 1991, pages 439-447, XP002969016 ISSN: 0022-2623
- VINCENT M ET AL: "SYNTHESIS AND ACE INHIBITORY ACTIVITY OF THE STEREOISOMERS OF PERINDOPRIL (S 9490) AND PERINDOPRILATE (S 9780)" DRUG DESIGN AND DISCOVERY, HARWOOD ACADEMIC PUBLISHERS GMBH, XX, vol. 9, no. 1, 1992, pages 11-28, XP000885876 ISSN: 1055-9612

## Description

La présente invention concerne un procédé de synthèse de la N-[(S)-1-carbéthoxybutyl]-(S)-alanine, et son application à la synthèse du perindopril et de ses sels pharmaceutiquement acceptables.

Plus spécifiquement, la présente invention concerne un nouveau procédé de synthèse du composé de formule (I) : 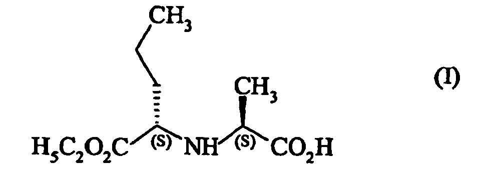 ainsi que ses sels d'addition à un acide ou à une base minéral(e) ou organique.

Le composé de formule (I) obtenu selon le procédé de l'invention est utile dans la synthèse du perindopril de formule (II) : 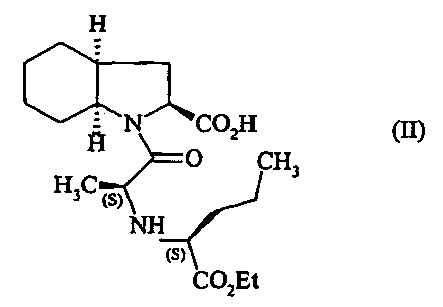 ainsi que dans celle de ses sels pharmaceutiquement acceptables.

Le perindopril, ainsi que ses sels, possèdent des propriétés pharmacologiques intéressantes. Leur principale propriété est d'inhiber l'enzyme de conversion de l'angiotensine I (ou kininase II), ce qui permet d'une part d'empêcher la transformation du décapeptide angiotensine I en octapeptide angiotensine II (vasoconstricteur), et d'autre part de prévenir la dégradation de la bradykinine (vasodilatateur) en peptide inactif.

Ces deux actions contribuent aux effets bénéfiques du perindopril dans les maladies cardiovasculaires, tout particulièrement l'hypertension artérielle et l'insuffisance cardiaque.

Le perindopril, sa préparation et son utilisation en thérapeutique ont été décrits dans le brevet européen EP 0 049 658.

Compte-tenu de l'intérêt pharmaceutique de ce composé, il était important de pouvoir accéder à l'intermédiaire de formule (I) avec un procédé de synthèse performant.

Quelques méthodes de préparation du composé de formule (I) sont déjà connues.
- Le journal Tet. Lett. 1982, 23 (16), 1677-80 décrit l'obtention du composé de formule (I) par réaction dans l'éthanol du 2-oxovalérate d'éthyle avec l'ester tert-butylique de l'alanine en présence de cyanoborohydrure de sodium.
- Le brevet EP 0 309 324 décrit l'accès au composé de formule (I) par réaction dans le diméthylformamide de l'ester benzylique de l'alanine avec l'α-bromovalérate d'éthyle en présence de triéthylamine.
- Les brevets EP 0 308 340 et EP 0 308 341 décrivent l'accès au composé de formule (I) par réaction dans l'eau du chlorhydrate de norvalinate d'éthyle avec l'acide pyruvique en présence d'hydrogène, de charbon palladié et de soude.

La demanderesse a présentement mis au point un nouveau procédé de synthèse du composé de formule (I).

Plus spécifiquement, la présente invention concerne un procédé de synthèse du composé de formule (I), caractérisé en ce que l'on met en réaction le glyoxylate d'éthyle de formule (III) : 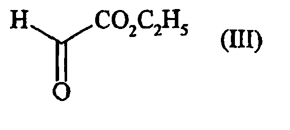 avec le composé de formule (IV) : 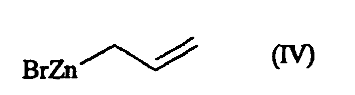 pour conduire au composé racémique de formule (V) : 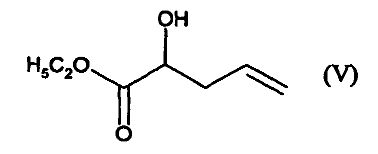 que l'on soumet à une déracémisation par hydrolyse enzymatique, pour conduire à l'isomère (R) de formule (Va) : 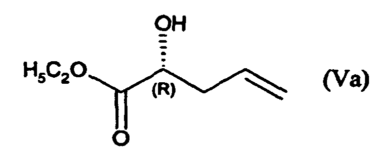 que l'on transforme en triflate de formule (VI) : 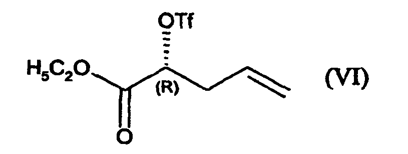 que l'on met en réaction avec le dérivé de l'alanine de formule (VII) : 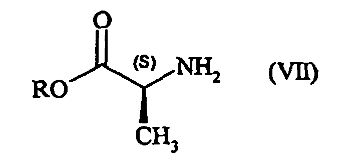
dans laquelle R représente un groupement alkyle (C₁-C₆) linéaire ou ramifié ou benzyle,
pour conduire au composé de formule (VIII) : 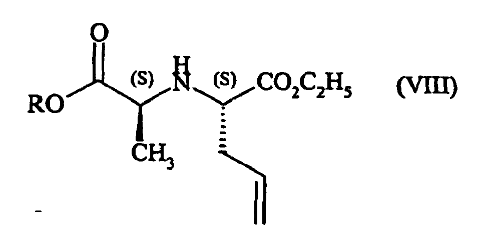
dans laquelle R est tel que défini précédemment,
que l'on soumet à une hydrogénation catalytique et à une déprotection de la fonction acide, pour conduire au composé de formule (I).

Les groupements R préférés sont les groupements tert-butyle et benzyle.

Le composé de formule (IV) est obtenu à partir de bromure d'allyle et de zinc en poudre et peut avantageusement être préparé *in situ*.

Le composé de formule (VI) est un produit nouveau, utile comme intermédiaire de synthèse dans l'industrie chimique ou pharmaceutique, notamment dans la synthèse du perindopril, et fait à ce titre partie intégrante de la présente invention.

### Exemple : N-[(S)-Carbéthoxy-1-butyl]-(S)-alanine

### Stade A : (2RS)-2-Hydroxy-4-penténoate d'éthyle

A une solution de 32,2 g de glyoxylate d'éthyle dans la diméthylformamide sont ajoutés successivement 28,4 ml de bromure d'allyle et 22,3 g de zinc, en maintenant la température du milieu réactionnel en-dessous de 50°C.
Le mélange réactionnel est ensuite ramené à température ambiante. Après 2h d'agitation à cette température, une solution aqueuse saturée en chlorure d'ammonium est ajoutée, le mélange est extrait et les phases organiques sont séchées, filtrées et évaporées, pour conduire au produit du titre.

### Stade B : (2R)-2-Hydroxy-4-penténoate d'éthyle

A 100 ml d'eau et 100 ml de tampon phosphate à pH=7 sont additionnés 53 g du composé racémique obtenu au stade précédent. Le mélange est ensuite agité pendant quelques minutes, puis le pH est ajusté à 7 par addition d'une solution d'hydroxyde de sodium 0,1 N, et 800 mg de lipase Pseudomonas Fluorescens à 31,5 u/mg sont ajoutés. L'hydrolyse a lieu à température ambiante, sous agitation et à pH constant, maintenu à 7 par addition d'une solution d'hydroxyde de sodium 1 N. L'hydrolyse est arrêtée quand 196 ml de la solution d'hydroxyde de sodium 1 N ont été ajoutés, ce qui correspond à un taux de conversion de 54%.
Le mélange est alors extrait à l'éther, les phases organiques sont séchées, filtrées et les solvants évaporés, pour conduire au composé du titre.

### Stade C : (2R)-2-(p-Trifluorométhanesulfonyloxy)-4-penténoate d'éthyle

A une solution à 0°C de 18,9 ml d'anhydride trifluorométhanesulfonique dans le dichlorométhane, on ajoute au goutte à goutte 14,6 g du composé obtenu au stade précédent et 9,7 ml de pyridine en solution dans le dichlorométhane. L'agitation est maintenue 2 h à 0°C, puis de l'eau est ajoutée. La phase organique est alors lavée avec une solution 1N d'acide chlorhydrique, une solution aqueuse saturée d'hydrogénocarbonate de sodium et de l'eau, puis la phase organique est séchée, filtrée, et les solvants sont évaporés, pour conduire au produit du titre.

### Stade D : (2S)-2-{[(1S)-2-Benzyloxy-1-méthyl-2-oxoéthyl]amino}-4-penténoate d'éthyle:

A une solution de 9 g de (S)-alaninate de benzyle dans le dichlorométhane sont ajoutés 10,2 ml de triéthylamine. Après 5 mn d'agitation, le mélange est refroidi à 0°C, et une solution de 13,8 g du composé obtenu au stade précédent dans le dichlorométhane est additionnée au goutte à goutte. Après 3 heures d'agitation à 0°C, le mélange réactionnel est ramené à température ambiante et agité 2 heures supplémentaires, puis une solution aqueuse saturée en chlorure d'ammonium est ajoutée. Le mélange est extrait à l'éther, la phase organique est séchée, filtrée puis évaporée, et le résidu obtenu purifié, pour conduire au produit du titre.

### Stade E: N-[(S)-Carbéthoxy-1-butyl]-(S)-alanine

Dans un hydrogénateur, placer 20 g du composé obtenu dans le stade précédent en solution dans l'éthanol, puis 0,5 g de Pd/C à 10 %. Hydrogéner sous pression normale et température ambiante, jusqu'à absorption de la quantité théorique d'hydrogène.
Eliminer le catalyseur par filtration, puis isoler la N-[(S)-carbéthoxy-1-butyl]-(S)-alanine par mise à sec.

## Revendications

1. Procédé de synthèse du composé de formule (I) : 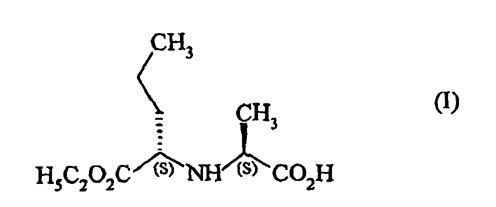
**caractérisé en ce que** l'on met en réaction le glyoxylate d'éthyle de formule (III) : 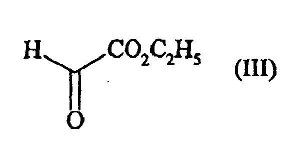
avec le composé de formule (IV) : 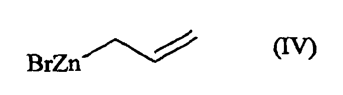
pour conduire au composé racémique de formule (V) : 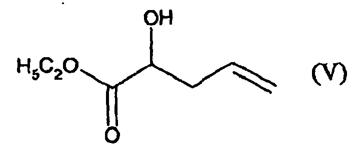
que l'on soumet à une déracémisation par hydrolyse enzymatique,
pour conduire à l'isomère (R) de formule (Va) : 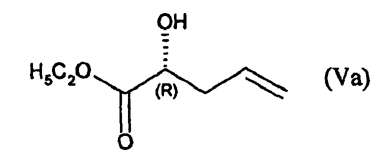
que l'on transforme en triflate de formule (VI) : 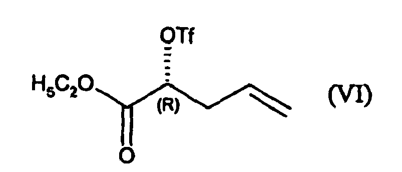
que l'on met en réaction avec le dérivé de l'alanine de formule (VII) : 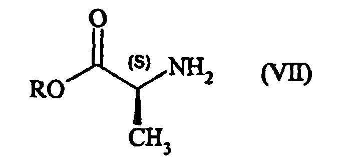
dans laquelle R représente un groupement alkyle (C₁-C₆) linéaire ou ramifié ou benzyle,
pour conduire au composé de formule (VIII) : 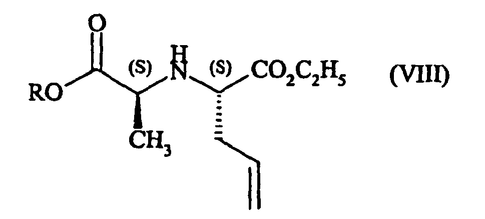
dans laquelle R est tel que défini précédemment,
que l'on soumet à une hydrogénation catalytique et à une déprotection de la fonction acide, pour conduire au composé de formule (I).

2. Procédé selon la revendication 1, **caractérisé en ce que** R représente un groupement benzyle ou tert-butyle.

3. Composé de formule (VI) :

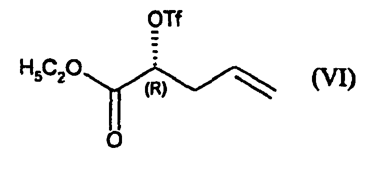

4. Procédé de synthèse du perindopril ou de ses sels pharmaceutiquement acceptables à partir d'un composé de formule (I), **caractérisé en ce que** ledit composé de formule (I) est obtenu selon le procédé de la revendication 1.

## Patentansprüche

1. Verfahren zur Synthese der Verbindung der Formel (I): 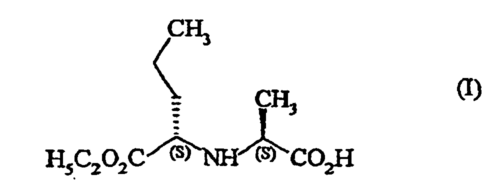
**dadurch gekennzeichnet, daß** man Glyoxylethylester der Formel (III): 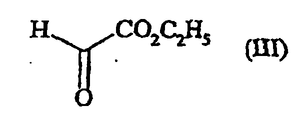
mit der Verbindung der Formel (IV): 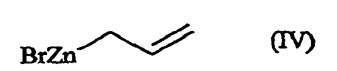
umsetzt, so daß man die racemische Verbindung der Formel (V) erhält: 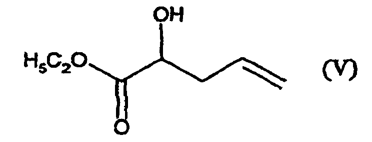
welche man einer Deracemisierung durch enzymatische Hydrolyse unterwirft, so daß man das Isomere (R) der Formel (Va) erhält: 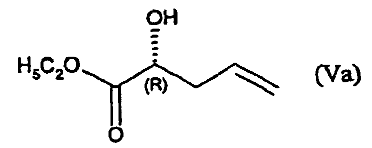
welches man in sein Triflat der Formel (VI) umwandelt: 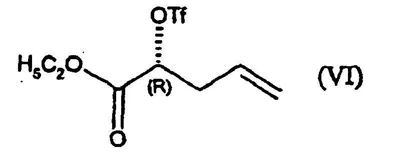
welches man mit dem Alaninderivat der Formel (VII): 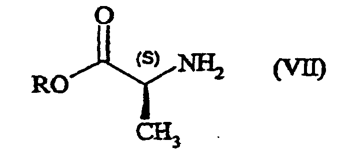
in der R eine geradkettige oder verzweigte (C₁-C₆)-Allkylgruppe oder eine Benzylgruppe bedeutet, umsetzt,
zur Bildung der Verbindung der Formel (VIII): 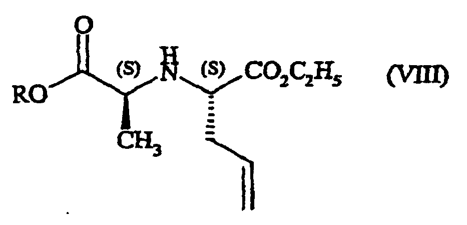
in der R die oben angegebenen Bedeutungen besitzt,
welche man einer katalytischen Hydrierung und einer Abspaltung der Schutzgruppe der Säurefunktion unterwirft zur Bildung der Verbindung der Formel (I).

2. Verfahren nach Anspruch 1, **dadurch gekennzeichnet, daß** R eine Benzylgruppe oder eine tert.-Butylgruppe bedeutet,

3. Verbindung der Formel (VI):

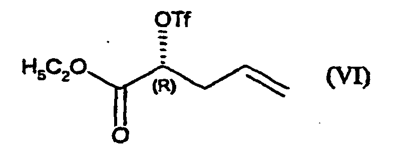

4. Verfahren zur Synthese von Perindopril oder seiner pharmazeutisch annehmbaren Salze ausgehend von einer Verbindung der Formel (I), **dadurch gekennzeichnet, daß** man die Verbindung der Formel (I) nach dem Verfahren gemäß Anspruch 1 herstellt.

## Claims

1. Process for the synthesis of the compound of formula (I) : 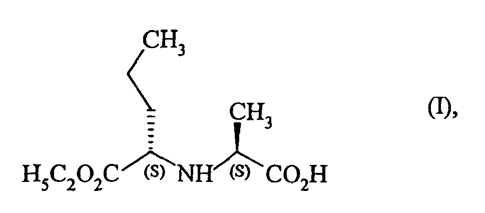
**characterised in that** ethyl glyoxylate of formula (III) : 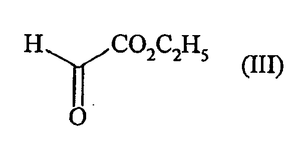
is reacted with the compound of formula (IV) : 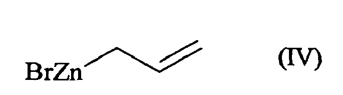
to yield the racemic compound of formula (V) : 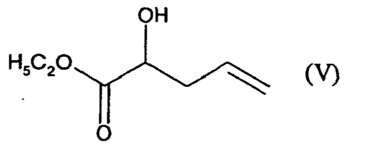
which is subjected to deracemisation by enzymatic hydrolysis
to yield the (R) isomer of formula (Va) : 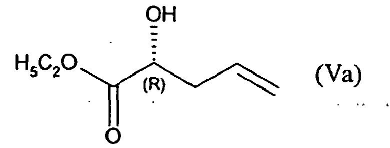
which is converted into the triflate of formula (VI) : 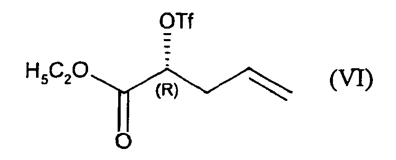
which is reacted with the alanine compound of formula (VII) : 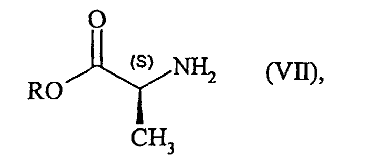
wherein R represents a linear or branched (C₁-C₆)alkyl or benzyl group,
to yield the compound of formula (VIII) : 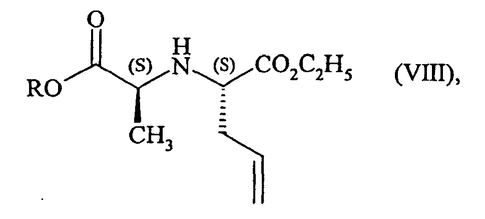
wherein R is as defined hereinbefore,
which is subjected to catalytic hydrogenation and to deprotection of the acid function to yield the compound of formula (I).

2. Process according to claim 1, **characterised in that** R represents a benzyl or tert-butyl group.

3. Compound of formula (VI) :

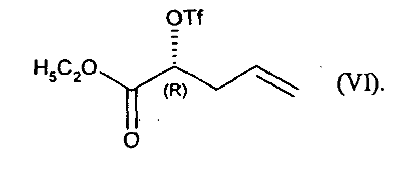

4. Process for the synthesis of perindopril or pharmaceutically acceptable salts thereof starting from a compound of formula (I), **characterised in that** the said compound of formula (I) is obtained according to the process of claim 1.
